# EUROPEAN PATENT APPLICATION

(11) **EP 3 719 496 A1**
(43) Date of publication of application: **07.10.2020**
(21) Application number: 18882494.0
(22) Date of filing: 28.11.2018
(51) Int. Cl.: G01N 33/48, C12M 1/34, G01N 33/50, G01N 33/53, C07K 14/805

(54) **METHOD FOR STABILIZING COMPLEX OF HEMOGLOBIN AND HAPTOGLOBIN AND A PRESERVATION SOLUTION FOR PRESERVING SPECIMENS CONTAINING HEMOGLOBIN**

(30) Priority: 01.12.2017 JP 2017231564
(71) Applicant: Eiken Kagaku Kabushiki Kaisha, Taito-ku Tokyo 110-8408 (JP)
(72) Inventor: YASUI Ryota, Shimotsuga-gun Tochigi 329-0114 (JP); SAKAMAKI Nozomi, Shimotsuga-gun Tochigi 329-0114 (JP)
(74) Representative: Boult Wade Tennant LLP
(86) International application number: PCT/JP2018/043791
(87) International publication number: WO 2019/107414

(57) **Abstract**

A method for stabilizing a hemoglobin-haptoglobin complex according to the present invention comprises: preserving the hemoglobin-haptoglobin complex in the presence of a degradation product of hemoglobin. According to such a method, the hemoglobin-haptoglobin complex can be stabilized.

## Description

### Technical Field

The present invention relates to a method for stabilizing a hemoglobin-haptoglobin complex, a preservation solution for preserving a hemoglobin-haptoglobin complex, a preservation solution for preserving a specimen containing hemoglobin, and a method and a kit for detecting hemoglobin in a specimen.

### Background Art

Detection of blood contained in feces, urine, saliva, and the like is useful for diagnosis of many diseases. For example, a fecal occult blood test which involves detecting blood in feces is used for screening for colorectal cancer. An immunological method which involves detecting hemoglobin contained in occult blood in a specimen such as feces using an anti-hemoglobin antibody is known as a method for detecting occult blood. A specimen to be subjected for an occult blood test is usually collected by a subject in a container containing a preservation solution, and is sent to an inspection institution such as a hospital. In many cases, a preservation solution (sample) containing a specimen is stored for some days before it is actually subjected to a test, and during that period, it is often placed under high temperature. Hemoglobin is unstable in a solution, and is particularly easily denatured or degraded under high temperature conditions. When a structure of an epitope or a surrounding site thereof changes due to denaturation or degradation of hemoglobin, an antibody cannot react with hemoglobin, and accordingly, the accuracy of detection of hemoglobin by an immunological method decreases.

In addition, in an occult blood test, an automated clinical analyzer that can perform prompt and accurate analysis on a large number of samples is widely used for measuring the concentration of hemoglobin by an immunological method. In general, in measurement using an automated clinical analyzer, changes in the device and changes in reagents used for the measurement greatly affect measurement results, and therefore, calibration or an quality control is regularly performed for the automated clinical analyzer using a calibrator or a control containing a known concentration of a substance to be measured. The calibration of an automated clinical analyzer is performed by measuring a calibrator containing a known concentration of a substance to be measured and creating a calibration curve, and the quality control of an automated clinical analyzer is performed by measuring a control containing a known concentration of a substance to be measured and checking whether or not the measured value is within a predetermined range. However, hemoglobin is unstable in a solution, and when a structure of an epitope or a surrounding site thereof changes due to denaturation or degradation of hemoglobin contained in a calibrator or a control, an antibody cannot react with hemoglobin, and therefore, the calibration and the quality control of an automated clinical analyzer cannot be performed accurately, and accurate measurement cannot be performed.

Under such a background, various methods have been proposed for stabilizing hemoglobin in a sample. For example, a method that involves adding an antibacterial agent such as thimerosal and chlorhexidine (for example, Patent Literature 1), a method that involves adding non-human animal hemoglobin (for example, Patent Literature 2), a method that involves adding non-human animal serum (for example, Patent Literature 3), a method that involves adding a glycosidase-type lytic enzyme (for example, Patent Literature 4), a method that involves adding a water-soluble transition metal complex (for example, Patent Literature 5), a method that involves adding an enzymatic degradation product of hemoglobin (for example, Patent Literature 6), a method that involves adding sulfurous acid, disulfurous acid, or the like (for example, Patent Literature 7), a method that involves adding an organic acid such as malic acid (for example, Patent Literature 8), a method that involves adding iminocarboxylic acid (for example, Patent Literature 9), a method that involves adding glyoxylic acid (for example, Patent Literature 10), and a method that involves adding haloalkanesulfonic acid (for example, Patent Literature 11) have been proposed.

However, since hemoglobin is extremely unstable, even when these methods for stabilizing hemoglobin are used, the denaturation or degradation of hemoglobin is not sufficiently suppressed. On the other hand, a method that involves adding haptoglobin to stabilize hemoglobin is also known (for example, Patent Literature 12). Haptoglobin is a protein which is present in blood of a wide range of animals and plays a role of recovering hemoglobin released into blood due to hemolysis of red blood cells. It is known that haptoglobin rapidly binds to hemoglobin to form a stable hemoglobin-haptoglobin complex (Hb-Hp complex). By adding haptoglobin in advance to a preservation solution or the like to which a specimen such as feces is to be added, hemoglobin contained in the specimen can form a stable hemoglobin-haptoglobin complex when the specimen is added.

### Citation List

### Patent Literature

[Patent Literature 1] Japanese Unexamined Patent Publication No. S63-271160
[Patent Literature 2] Japanese Unexamined Patent Publication No. H2-296149
[Patent Literature 3] Japanese Unexamined Patent Publication No. H4-145366
[Patent Literature 4] Japanese Examined Patent Publication No. H5-69466
[Patent Literature 5] Japanese Unexamined Patent Publication No. H7-229902
[Patent Literature 6] Japanese Unexamined Patent Publication No. H11-218533
[Patent Literature 7] Japanese Unexamined Patent Publication No. 2000-258420
[Patent Literature 8] Japanese Unexamined Patent Publication No. 2003-14768
[Patent Literature 9] Japanese Unexamined Patent Publication No. 2009-097956
[Patent Literature 10] Japanese Unexamined Patent Publication No. 2013-257216
[Patent Literature 11] Japanese Unexamined Patent Publication No. 2016-191580
[Patent Literature 12] Japanese Unexamined Patent Publication No. H10-132824

### Summary of Invention

### Technical Problem

Since there are many bacteria or proteolytic enzymes which cause degradation of hemoglobin in a specimen, particularly in feces, derived from a living body, even a hemoglobin-haptoglobin complex is sometimes degraded. Therefore, an object of the present invention is to stabilize a hemoglobin-haptoglobin complex.

### Solution to Problem

A method for stabilizing a hemoglobin-haptoglobin complex according to the present invention comprises: preserving the hemoglobin-haptoglobin complex in the presence of a degradation product of hemoglobin. The degradation product of hemoglobin may be an enzymatic degradation product of hemoglobin. The above-described method may comprise preserving a hemoglobin-haptoglobin complex in a preservation solution comprising the degradation product of hemoglobin, and a concentration of the degradation product of hemoglobin in the preservation solution in terms of iron equivalent may be 0.012 mg/L or more. The hemoglobin-haptoglobin complex may comprise a hemoglobin-haptoglobin complex formed by bringing a specimen comprising hemoglobin into contact with haptoglobin. The specimen may be feces, saliva, or urine, or may be feces.

A preservation solution for preserving a hemoglobin-haptoglobin complex according to the present invention comprises: a degradation product of hemoglobin. The preservation solution may further comprise the hemoglobin-haptoglobin complex, and the preservation solution may be used as a calibrator or a control.

A preservation solution for preserving a specimen comprising hemoglobin according to the present invention comprises: haptoglobin; and a degradation product of hemoglobin. The specimen may be feces, saliva, or urine.

The degradation product of hemoglobin may be an enzymatic degradation product of hemoglobin. The concentration of the degradation product of hemoglobin in terms of iron equivalent may be 0.012 mg/L or more.

A method for detecting hemoglobin in a specimen according to the present invention comprises: adding a specimen to the above-described preservation solution for preserving a specimen comprising hemoglobin to obtain a sample comprising the specimen; and detecting hemoglobin in the sample by an immunological method, wherein hemoglobin in the sample is forming a complex with haptoglobin.

A kit for detecting hemoglobin in a specimen according to the present invention comprises: the above-described preservation solution for preserving a specimen comprising hemoglobin; and a reagent comprising an anti-hemoglobin antibody.

### Advantageous Effects of Invention

According to the present invention, the hemoglobin-haptoglobin complex can be stabilized. In other words, according to the present invention, denaturation and degradation of hemoglobin in the hemoglobin-haptoglobin complex can be suppressed. Therefore, according to the present invention, hemoglobin in a specimen can be detected by an immunological method with higher accuracy. In addition, a calibrator or a control having excellent storage stability can be provided.

### Brief Description of Drawings

Fig. 1 is a graph showing an effect of addition of a degradation product of hemoglobin on a recovery rate of a hemoglobin-haptoglobin complex at 37°C.
Fig. 2 is a graph showing an effect of addition of a degradation product of hemoglobin on a recovery rate of a hemoglobin-haptoglobin complex at 56°C.
Fig. 3 is a graph showing an effect of addition of a degradation product of hemoglobin on a recovery rate of hemoglobin at 37°C.
Fig. 4 is a graph showing an effect of addition of a degradation product of hemoglobin on a recovery rate of hemoglobin at 56°C.
Fig. 5 is a graph showing a relationship between a concentration of a degradation product of hemoglobin and a recovery rate of hemoglobin in feces.
Fig. 6 is a graph showing a relationship between a concentration of a degradation product of hemoglobin and a recovery rate of hemoglobin in feces.
Fig. 7 is a graph showing a recovery rate of hemoglobin in feces in a case where haptoglobin is added, but no degradation product of hemoglobin is added.
Fig. 8 is a graph showing a recovery rate of hemoglobin in a case where haptoglobin and a degradation product of hemoglobin are added.
Fig. 9 is a graph showing a recovery rate of hemoglobin in a case where haptoglobin and a degradation product of hemoglobin are not added.
Fig. 10 is a graph showing a recovery rate of hemoglobin in a case where no haptoglobin is added, but a degradation product of hemoglobin is added.

### Description of Embodiments

A method for stabilizing a hemoglobin-haptoglobin complex according to the present invention comprise: preserving the hemoglobin-haptoglobin complex in the presence of a degradation product of hemoglobin.

The degradation product of hemoglobin is a fragmented hemoglobin, and examples of a fragmentation method include methods such as an enzymatic degradation method and a chemical degradation method. The degradation product of hemoglobin is preferably an enzymatic degradation product of hemoglobin which has been conventionally used. The enzyme may be a proteolytic enzyme such as trypsin, pepsin, and Alcalase. The degradation product of hemoglobin may be completely degraded hemoglobin, partially degraded hemoglobin, or a mixture thereof. The completely degraded hemoglobin means a degradation product of hemoglobin obtained when an enzymatic degradation reaction is completed, or the same degradation product of hemoglobin but obtained by a chemical degradation method. The partially degraded hemoglobin means a degradation product of hemoglobin obtained at an arbitrary stage before an enzymatic degradation reaction is completed, or the same degradation product of hemoglobin but obtained by a chemical degradation method. Partially degraded hemoglobin is preferable as a degradation product of hemoglobin. That is, an enzymatically partially degraded product of hemoglobin is preferable as a degradation product of hemoglobin. Partially degraded hemoglobin has excellent solubility, and an auxiliary stabilizing effect of a hemoglobin-haptoglobin complex due to globin fragments can be expected. It is preferable that a degradation product of hemoglobin comprises heme, which is a complex of iron and porphyrin, as well as globin degraded to a degree such that it does not exhibit antigenicity. Furthermore, a degradation product of hemoglobin is preferably degraded to a degree such that the degradation product of hemoglobin does not form a complex with haptoglobin. Animals from which a degradation product of hemoglobin is derived are not limited, and examples thereof may include humans or vertebrates other than humans having hemoglobin, or may be mammals such as pigs, cattle, horses, sheep, goats, and rabbits, birds, or fishes.

An aspect of the present method comprises preserving a hemoglobin-haptoglobin complex in a preservation solution comprising a degradation product of hemoglobin.

The preservation solution may be a buffer solution comprising a good buffer agent such as 2-morpholinoethanesulfonic acid (MES), hydroxyethylpiperazine-2-ethanesulfonic acid (HEPES), or piperazine-bis(2-ethanesulfonic acid) (PIPES), or may be a phosphate buffer solution, a tris buffer solution, a glycine buffer solution, or the like.

The concentration of a degradation product of hemoglobin in terms of iron equivalent is preferably 0.012 mg/L or more, 0.012 mg/L to 60 mg/L, 0.12 mg/L to 12 mg/L, 1.2 mg/L to 6.3 mg/L, or 1.2 mg/L to 3.6 mg/L. When the concentration of a degradation product of hemoglobin in terms of iron equivalent is 60 mg/L or less, the viscosity of a preservation solution does not become excessively high, and therefore, the concentration of hemoglobin or a hemoglobin-haptoglobin complex in a sample is easily measured. In addition, when the concentration of a degradation product of hemoglobin in terms of iron equivalent is 60 mg/L or less, coloration of a preservation solution due to the degradation product of hemoglobin can be suppressed. Iron equivalent means an amount (mg Fe/L) of iron atoms contained in a degradation product of hemoglobin. The iron equivalent amount of a degradation product of hemoglobin may be measured by ortho-phenanthroline colorimetry, an atomic absorption method, or the like.

The pH of a preservation solution may be 5 to 10, or 6 to 8.

Known additives, for example, antibacterial agents such as sodium azide (NaN₃), pH adjusting agents, and salts for adjusting ionic strength, which may be used when preserving hemoglobin may be further added to a preservation solution. An antibacterial agent includes antibiotics and lytic enzymes. Examples of additives include known components, for example, amino acids such as lysine and histidine, albumin, a protease inhibitor, a water-soluble complex of transition metal ions, and ethylenediamine tetraacetic acid (EDTA), which are known to have a stabilizing effect on hemoglobin. Examples of albumin include serum albumin such as bovine serum albumin (BSA) and albumin (ovalbumin) derived from egg white.

By further adding a known concentration of a hemoglobin-haptoglobin complex to the preservation solution having the above-described composition, the above-described preservation solution may be used as a calibrator or a control for detecting or analyzing the hemoglobin-haptoglobin complex. In such a calibrator or control, the hemoglobin-haptoglobin complex is stabilized by a degradation product of hemoglobin, and therefore, can be stably preserved even under high temperature conditions.

A more specific aspect of the present method comprises preserving a hemoglobin-haptoglobin complex formed by bringing a specimen containing hemoglobin into contact with haptoglobin in the above-described preservation solution. The specimen containing hemoglobin may be feces, saliva, or urine. Since there are particularly many bacteria or proteolytic enzymes which cause degradation of hemoglobin in feces, the method of the present invention is particularly effective.

The specimen containing hemoglobin may be brought into contact with haptoglobin in any manner. Preferably, the specimen containing hemoglobin may be added to the above-described preservation solution further comprising haptoglobin. Hemoglobin in a specimen reacts quickly with haptoglobin in a preservation solution to form a hemoglobin-haptoglobin complex. By preserving the specimen in the preservation solution as it is, the hemoglobin-haptoglobin complex can be stably preserved. In other words, according to the above-described method for stabilizing a hemoglobin-haptoglobin complex, the specimen can be preserved while maintaining the structure of an epitope of hemoglobin and a surrounding site thereof in the hemoglobin-haptoglobin complex. Accordingly, it can also be said that the present invention provides a preservation solution for preserving a specimen comprising hemoglobin. When hemoglobin forms a complex with haptoglobin, hemoglobin is dissociated from a tetramer (α2β2) in which two α chains and two β chains are assembled into two dimers (αβ). However, this phenomenon does not correspond to the "degradation" and "denaturation" in the present specification.

In the present specification, haptoglobin is not particularly limited as long as it combines with hemoglobin to form a hemoglobin-haptoglobin complex. Since species specificity of the binding of hemoglobin to haptoglobin is low, haptoglobin derived from a wide range of species may be used. When hemoglobin in a specimen is human hemoglobin, haptoglobin derived from humans and animals such as horses, pigs, monkeys, dogs, rabbits, and rats may be used. The haptoglobin does not necessarily have to be highly purified.

The preservation solution for preserving a specimen comprising hemoglobin according to the present invention is obtained by further adding haptoglobin to the above-described preservation solution comprising a degradation product of hemoglobin. The concentration of haptoglobin in the preservation solution depends on the amount of specimen, and examples thereof include 0.05 unit/L to 50 unit/L, 0.1 unit/L to 10 unit/L, or 0.2 unit/L to 2 unit/L. Here, one unit represents an amount of haptoglobin binding to 1 mg of hemoglobin. The concentration of haptoglobin is preferably adjusted to a concentration sufficient for making all hemoglobin in a specimen form a complex with haptoglobin.

According to the above-described stabilization method or preservation solution, hemoglobin in a specimen can be stabilized in a form of a hemoglobin-haptoglobin complex. In other words, according to the above-described method or preservation solution, denaturation and degradation of hemoglobin in a specimen can be suppressed, and accordingly, the structure of an epitope of hemoglobin and a surrounding site thereof can be maintained. Accordingly, when hemoglobin in a specimen is detected by an immunological method, the accuracy of the detection is expected to improve.

The method for detecting hemoglobin in a specimen provided by the present invention comprises: adding a specimen to the above-described preservation solution for preserving a specimen comprising hemoglobin to obtain a sample comprising the specimen; and detecting hemoglobin in the sample by an immunological method.

The immunological method is a method utilizing an anti-hemoglobin antibody, and a known immunological method may be used. The immunological method may be, for example, an immunoagglutination method (for example, a latex agglutination method or a colloidal gold agglutination method), an immunochromatography, or an ELISA method.

The detection of hemoglobin in a specimen may be performed, for example, as follows. First, a specimen is collected in a container comprising a preservation solution. In a case where there is hemoglobin in the specimen, the hemoglobin forms a hemoglobin-haptoglobin complex. Not all hemoglobin in the specimen necessarily forms a complex, and hemoglobin which does not form a complex with haptoglobin may be present in the preservation solution (sample) comprising the specimen. However, it is preferable that substantially all hemoglobin in the specimen form a complex with haptoglobin. After the specimen in the container is preserved for an arbitrary time, the preservation solution comprising the specimen is filtered. Next, hemoglobin in the filtrate is detected by a latex agglutination method. More specifically, a reagent comprising an anti-hemoglobin antibody with latex particles bound to its surface is added to the filtrate. Preferably, the anti-hemoglobin antibody can react with the epitope of hemoglobin in the hemoglobin-haptoglobin complex, and does not cross-react with haptoglobin. If hemoglobin is present in the filtrate, the anti-hemoglobin antibody reacts with the hemoglobin, and latex particles bound to the antibody agglutinate. The change in turbidity due to the agglutination is measured, and the concentration of hemoglobin in the filtrate is obtained using a calibration curve created using a calibrator comprising a hemoglobin-haptoglobin complex with a known hemoglobin concentration. In addition, the concentration of the hemoglobin-haptoglobin complex in the filtrate may also be obtained using the calibration curve created based on the concentration of the hemoglobin-haptoglobin complex of the calibrator.

The present invention also provides a sample that may be used for detecting hemoglobin in a specimen. The sample comprises a hemoglobin-haptoglobin complex and a degradation product of hemoglobin. More specifically, the sample comprises a degradation product of hemoglobin and a hemoglobin-haptoglobin complex formed by haptoglobin and hemoglobin in the specimen. Since the hemoglobin-haptoglobin complex is stabilized in the sample, hemoglobin in the specimen can be detected with higher accuracy.

The present invention further provides a kit that may be used when detecting hemoglobin in a specimen by the above-described method. The kit comprises: the above-described preservation solution for preserving a specimen comprising hemoglobin; and a reagent comprising an anti-hemoglobin antibody. There is no limitation on the anti-hemoglobin antibody, and the anti-hemoglobin antibody may be a polyclonal antibody, a monoclonal antibody, or a fragment of an anti-hemoglobin antibody that can react with hemoglobin. A substance such as latex necessary for detection may be bound to an anti-hemoglobin antibody. The kit may further comprise arbitrary components such as a tool and a container for collecting a specimen, a calibrator, a control, and a solution for diluting a specimen.

### [Examples]

### (Test Example 1-1)

Preservation solutions to which 50 mM HEPES (pH 7.4), 0.1% BSA, 0.1% NaN₃, and a 0 to 5,000 mg/L (0 to 60 mg Fe/L of iron equivalent amount) degradation product of hemoglobin (Hb degradation product) were added were prepared. A degradation product of hemoglobin (manufactured by ILS Inc.) derived from a pig, obtained using a proteolytic enzyme, was used as the degradation product of hemoglobin. The degradation product of hemoglobin was analyzed by SDS-PAGE, and a broad band was observed at a position of a molecular weight of 3 kDa to 9 kDa. The average molecular weight of the degradation product of hemoglobin estimated from the content of iron was 4.6 kDa. The degradation product of hemoglobin was used after confirming that the degradation product of hemoglobin was degraded to a degree such that hemoglobin did not form a complex with haptoglobin. A hemoglobin-haptoglobin complex (containing about 900 µg/L hemoglobin and about 0.9 unit/L haptoglobin as constituents) was added to each of the preservation solutions, and the preservation solutions were preserved at 4°C, 25°C, 37°C, 45°C, or 56°C for 0, 3, 7, 12, and 20 days. The concentrations (µg/L) of the Hb-Hp complexes in the preserved samples were measured by a latex agglutination method. The concentrations of the Hb-Hp complexes were obtained in terms of the content of hemoglobin in the Hb-Hp complexes.

The concentrations of the Hb-Hp complexes were measured using a measurement reagent (OC-Hemodia (registered trademark) Auto S 'Eiken'" (manufactured by Eiken Chemical Co., Ltd.) and a measurement device "JCA-BM2250" (manufactured by JEOL Ltd.) The above-described measurement reagent contains anti-human hemoglobin rabbit polyclonal antibody immobilized latex particles.

The measurement conditions in the JCA-BM2250 are as follows.
Amount of sample: 7.0 µL
First reagent: 40 µL
Second reagent: 20 µL
Measurement wavelength: 658 nm

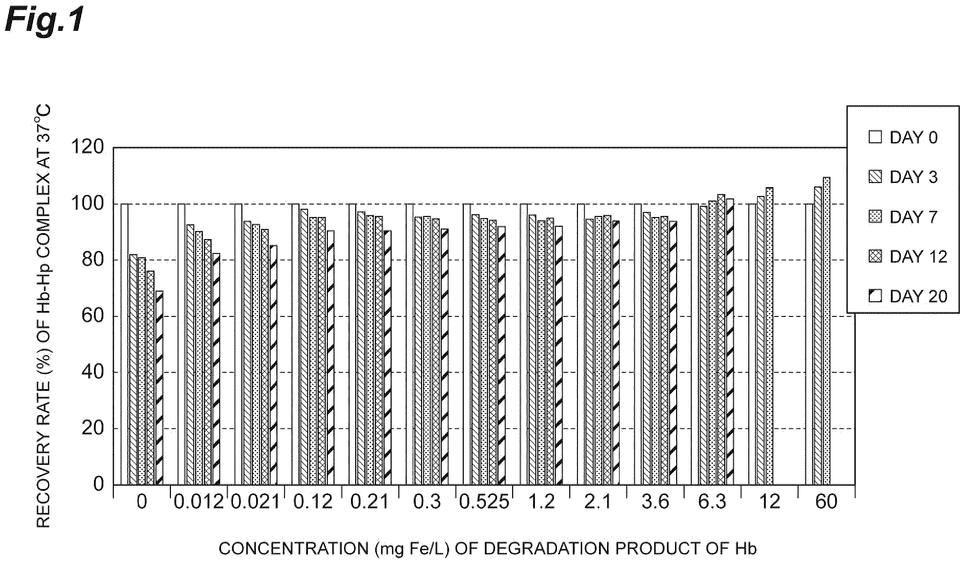

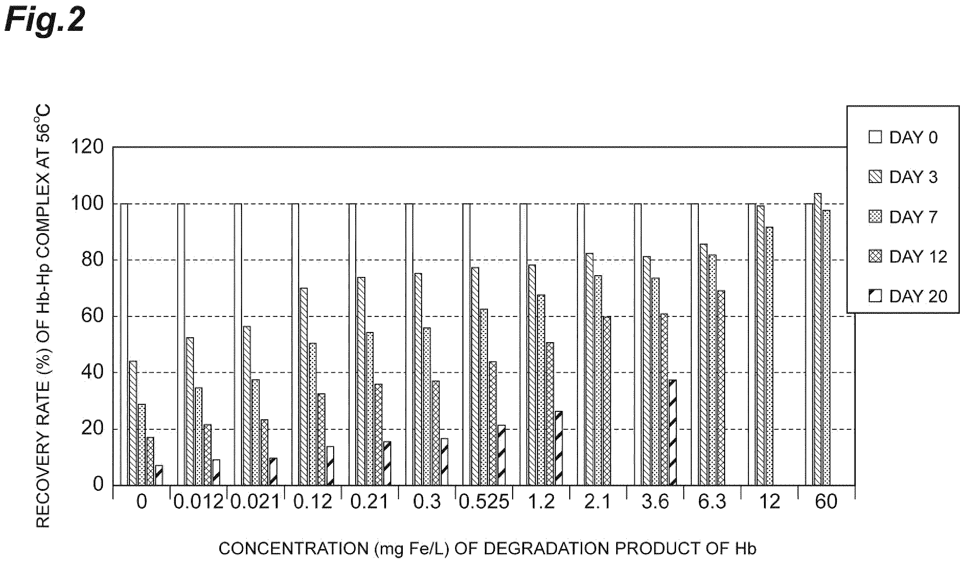

The recovery rates (%) of the Hb-Hp complexes were calculated from the measured concentrations of the Hb-Hp complexes, based on the concentrations of the Hb-Hp complexes immediately after the Hb-Hp complexes were added (that is, concentrations on day 0 after the addition of the Hb-Hp complexes). The results are shown in Table 1 and Figs. 1 and 2. In Table 1, the concentration of an Hb degradation product is expressed in terms of iron equivalent concentration (mg Fe/L). As shown in this table and these drawings, the recovery rates of the Hb-Hp complexes improved due to the addition of the Hb degradation product. The recovery rate on day 20 after the preservation at 37°C was 80% or more (Fig. 1), and the recovery rate on day 3 after the preservation at 56°C was 50% or more (Fig. 2). Thus, high preservation stability of the Hb-Hp complexes was achieved even in the preservation in a high temperature environment. In addition, the recovery rate (%) improved in accordance with the concentration of the degradation products of hemoglobin added. This result showed that the Hb degradation products stabilized the Hb-Hp complexes. Although test results in cases where the temperature was 25°C and 45°C are not shown, results from which the same conclusions as above can be drawn were obtained for these temperatures.

**[Table 1]**

| Preservation temperature | Concentration (mg Fe/L) of Hb degradation product * | Concentration (µg/L) of Hb-Hp complex ** | | | | | Recovery rate (%) of Hb-Hp complex | | | | |
|---|---|---|---|---|---|---|---|---|---|---|---|
| | | Day 0 | Day 3 | Day 7 | Day 12 | Day 20 | Day 0 | Day 3 | Day 7 | Day 12 | Day 20 |
| 4°C | 0 | 802 | 808 | 800 | 808 | 794 | 100 | 101 | 100 | 101 | 99 |
| | 0.012 | 835 | 838 | 841 | 848 | 828 | 100 | 100 | 101 | 102 | 99 |
| | 0.021 | 804 | 800 | 817 | 810 | 793 | 100 | 100 | 102 | 101 | 99 |
| | 0.12 | 733 | 737 | 742 | 737 | 721 | 100 | 101 | 101 | 101 | 98 |
| | 0.21 | 771 | 769 | 773 | 783 | 760 | 100 | 100 | 100 | 102 | 99 |
| | 0.3 | 822 | 822 | 829 | 821 | 815 | 100 | 100 | 101 | 100 | 99 |
| | 0.525 | 795 | 796 | 791 | 808 | 792 | 100 | 100 | 99 | 102 | 100 |
| | 1.2 | 794 | 794 | 795 | 799 | 793 | 100 | 100 | 100 | 101 | 100 |
| | 2.1 | 752 | 749 | 761 | 761 | 745 | 100 | 100 | 101 | 101 | 99 |
| | 3.6 | 724 | 732 | 740 | 736 | 724 | 100 | 101 | 102 | 102 | 100 |
| | 6.3 | 684 | 691 | 696 | 697 | 686 | 100 | 101 | 102 | 102 | 100 |
| | 12 | 739 | 749 | 749 | - | - | 100 | 101 | 101 | - | - |
| | 60 | 632 | 629 | 636 | - | - | 100 | 100 | 101 | - | - |
| 37°C | 0 | 802 | 657 | 648 | 610 | 553 | 100 | 82 | 81 | 76 | 69 |
| | 0.012 | 835 | 773 | 753 | 729 | 688 | 100 | 93 | 90 | 87 | 82 |
| | 0.021 | 804 | 754 | 745 | 731 | 685 | 100 | 94 | 93 | 91 | 85 |
| | 0.12 | 733 | 719 | 698 | 698 | 663 | 100 | 98 | 95 | 95 | 90 |
| | 0.21 | 771 | 749 | 739 | 737 | 697 | 100 | 97 | 96 | 96 | 90 |
| | 0.3 | 822 | 783 | 786 | 778 | 749 | 100 | 95 | 96 | 95 | 91 |
| | 0.525 | 795 | 764 | 754 | 749 | 731 | 100 | 96 | 95 | 94 | 92 |
| | 1.2 | 794 | 763 | 746 | 754 | 731 | 100 | 96 | 94 | 95 | 92 |
| | 2.1 | 752 | 711 | 719 | 721 | 706 | 100 | 95 | 96 | 96 | 94 |
| | 3.6 | 724 | 702 | 689 | 692 | 679 | 100 | 97 | 95 | 96 | 94 |
| | 6.3 | 684 | 678 | 691 | 707 | 696 | 100 | 99 | 101 | 103 | 102 |
| | 12 | 739 | 759 | 781 | - | - | 100 | 103 | 106 | - | - |
| | 60 | 632 | 669 | 691 | - | - | 100 | 106 | 109 | - | - |
| 56°C | 0 | 802 | 353 | 231 | 137 | 57 | 100 | 44 | 29 | 17 | 7 |
| | 0.012 | 835 | 437 | 289 | 180 | 76 | 100 | 52 | 35 | 22 | 9 |
| | 0.021 | 804 | 453 | 302 | 188 | 78 | 100 | 56 | 38 | 23 | 10 |
| | 0.12 | 733 | 513 | 369 | 238 | 101 | 100 | 70 | 50 | 32 | 14 |
| | 0.21 | 771 | 569 | 418 | 277 | 120 | 100 | 74 | 54 | 36 | 16 |
| | 0.3 | 822 | 618 | 459 | 305 | 137 | 100 | 75 | 56 | 37 | 17 |
| | 0.525 | 795 | 614 | 497 | 348 | 170 | 100 | 77 | 63 | 44 | 21 |
| | 1.2 | 794 | 621 | 536 | 402 | 209 | 100 | 78 | 68 | 51 | 26 |
| | 2.1 | 752 | 619 | 559 | 449 | - | 100 | 82 | 74 | 60 | - |
| | 3.6 | 724 | 588 | 532 | 440 | 271 | 100 | 81 | 73 | 61 | 37 |
| | 6.3 | 684 | 585 | 559 | 472 | - | 100 | 86 | 82 | 69 | - |
| | 12 | 739 | 733 | 677 | - | - | 100 | 99 | 92 | - | - |
| | 60 | 632 | 655 | 617 | - | - | 100 | 104 | 98 | - | - |

| | | | | | | | | | | | |
|---|---|---|---|---|---|---|---|---|---|---|---|
| *: Concentration in terms of iron equivalent **: Content of hemoglobin in Hb-Hp complex "-" in the table indicates that measurement was not performed. | | | | | | | | | | | |

### (Test Example 1-2)

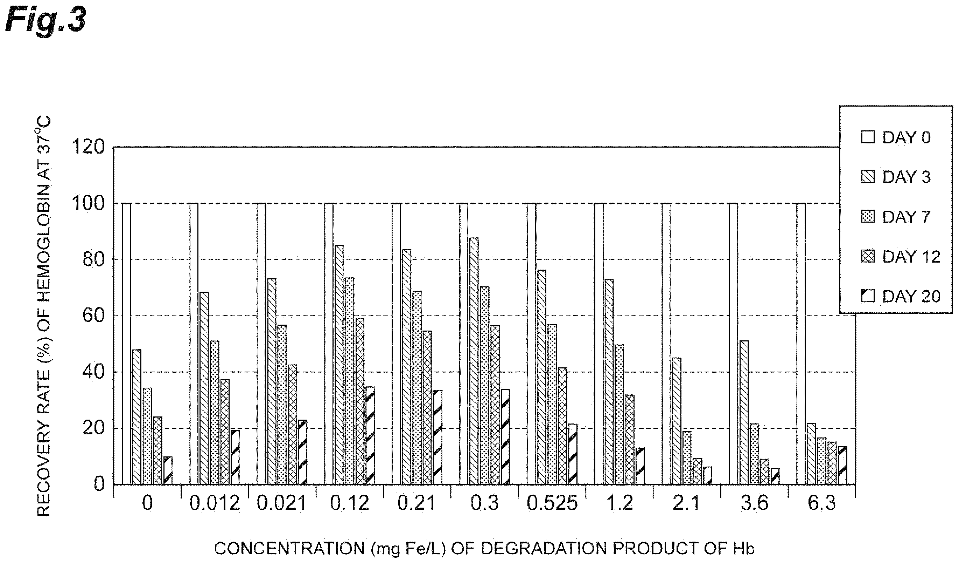

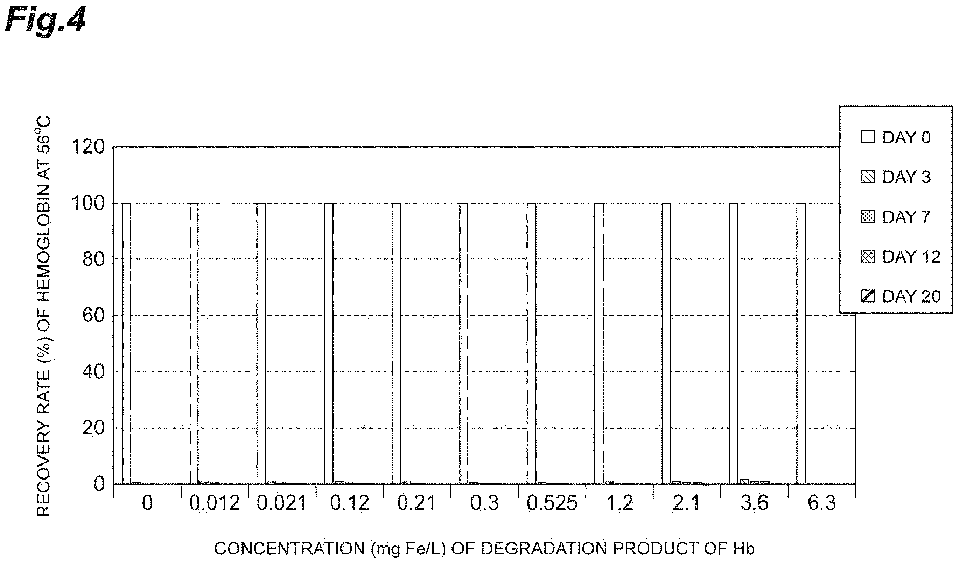

For reference, the same test as in Test Example 1-1 was performed, but with hemoglobin added to a preservation solution instead of an Hb-Hp complex. Results of the calculation of the recovery rates (%) of hemoglobin are shown in Table 2 and Figs. 3 and 4. As shown in this table and these drawings, although the recovery rates of hemoglobin improved due to the addition of the Hb degradation product, the recovery rates were low compared to the case of hemoglobin forming a complex with haptoglobin (Test Example 1-1). The maximum recovery rate on day 20 after the preservation at 37°C was 35% (Fig. 3), and the recovery rate on day 3 after the preservation at 56°C was 2% or low (Fig. 4). Thus, the preservation stability of hemoglobin in the preservation in a high temperature environment was significantly low.

**[Table 2]**

| Preservation temperature | Concentration (mg Fe/L) of Hb degradation product * | Concentration (µg/L) of hemoglobin | | | | | Recovery rate (%) of hemoglobin | | | | |
|---|---|---|---|---|---|---|---|---|---|---|---|
| | | Day 0 | Day 3 | Day 7 | Day 12 | Day 20 | Day 0 | Day 3 | Day 7 | Day 12 | Day 20 |
| 4°C | 0 | 957 | 944 | 930 | 931 | 886 | 100 | 99 | 97 | 97 | 93 |
| | 0.012 | 965 | 953 | 942 | 952 | 935 | 100 | 99 | 98 | 99 | 97 |
| | 0.021 | 954 | 933 | 930 | 936 | 910 | 100 | 98 | 97 | 98 | 95 |
| | 0.12 | 924 | 915 | 919 | 922 | 901 | 100 | 99 | 99 | 100 | 98 |
| | 0.21 | 955 | 959 | 936 | 933 | 923 | 100 | 100 | 98 | 98 | 97 |
| | 0.3 | 959 | 940 | 942 | 953 | 934 | 100 | 98 | 98 | 99 | 97 |
| | 0.525 | 923 | 904 | 898 | 900 | 882 | 100 | 98 | 97 | 98 | 96 |
| | 1.2 | 957 | 931 | 941 | 940 | 929 | 100 | 97 | 98 | 98 | 97 |
| | 2.1 | 871 | 861 | 853 | 852 | 832 | 100 | 99 | 98 | 98 | 96 |
| | 3.6 | 898 | 896 | 883 | 898 | 864 | 100 | 100 | 98 | 100 | 96 |
| | 6.3 | 742 | 732 | 729 | 703 | 667 | 100 | 99 | 98 | 95 | 90 |
| 37°C | 0 | 957 | 458 | 329 | 229 | 93 | 100 | 48 | 34 | 24 | 10 |
| | 0.012 | 965 | 660 | 491 | 360 | 186 | 100 | 68 | 51 | 37 | 19 |
| | 0.021 | 954 | 697 | 540 | 405 | 218 | 100 | 73 | 57 | 42 | 23 |
| | 0.12 | 924 | 786 | 678 | 545 | 321 | 100 | 85 | 73 | 59 | 35 |
| | 0.21 | 955 | 798 | 656 | 521 | 318 | 100 | 84 | 69 | 55 | 33 |
| | 0.3 | 959 | 840 | 675 | 541 | 323 | 100 | 88 | 70 | 56 | 34 |
| | 0.525 | 923 | 703 | 524 | 382 | 198 | 100 | 76 | 57 | 41 | 21 |
| | 1.2 | 957 | 697 | 474 | 304 | 124 | 100 | 73 | 50 | 32 | 13 |
| | 2.1 | 871 | 391 | 163 | 80 | 54 | 100 | 45 | 19 | 9 | 6 |
| | 3.6 | 898 | 458 | 194 | 80 | 50 | 100 | 51 | 22 | 9 | 6 |
| | 6.3 | 742 | 161 | 123 | 112 | 100 | 100 | 22 | 17 | 15 | 13 |
| 56°C | 0 | 957 | 2 | 0 | 0 | 0 | 100 | 0 | 0 | 0 | 0 |
| | 0.012 | 965 | 6 | 0 | 0 | 0 | 100 | 1 | 0 | 0 | 0 |
| | 0.021 | 954 | 7 | 3 | 0 | 0 | 100 | 1 | 0 | 0 | 0 |
| | 0.12 | 924 | 7 | 3 | 1 | 1 | 100 | 1 | 0 | 0 | 0 |
| | 0.21 | 955 | 8 | 3 | 1 | 1 | 100 | 1 | 0 | 0 | 0 |
| | 0.3 | 959 | 7 | 2 | 2 | 0 | 100 | 1 | 0 | 0 | 0 |
| | 0.525 | 923 | 5 | 2 | 1 | 0 | 100 | 1 | 0 | 0 | 0 |
| | 1.2 | 957 | 6 | 2 | 2 | 0 | 100 | 1 | 0 | 0 | 0 |
| | 2.1 | 871 | 6 | 0 | 1 | 0 | 100 | 1 | 0 | 0 | 0 |
| | 3.6 | 898 | 7 | 4 | 4 | -2 | 100 | 1 | 0 | 0 | 0 |
| | 6.3 | 742 | 12 | 7 | 7 | 2 | 100 | 2 | 1 | 1 | 0 |
| *: Concentration in terms of iron equivalent | | | | | | | | | | | |

### (Test Example 2-1)

Preservation solutions to which 50 mM HEPES (pH 6.8), 0.1% BSA, 0.1% NaN₃, 0 to 1,000 mg/L (0 to 12 mg Fe/L of iron equivalent amount) degradation product of hemoglobin (manufactured by ILS Inc.), and 1 unit/L haptoglobin were added were prepared. Fecal specimens to which hemoglobin was added were added to the preservation solutions so that the concentrations of feces became 0.5 mass%, and preserved at 37°C for 0, 7, and 14 days. The concentrations (µg/L) of hemoglobin in the preserved samples were measured by a latex agglutination method. Note that hemoglobin was added to the fecal specimens in such amounts that the concentrations of hemoglobin in the samples became about 500 µg/L.

The concentrations of hemoglobin were measured using a measurement reagent "OC-Hemodia (registered trademark) Auto III 'Eiken'" (manufactured by Eiken Chemical Co., Ltd.) and a measurement device "OC-Sensor DIANA" (manufactured by Eiken Chemical Co., Ltd.). The above-described measurement reagent contains anti-human hemoglobin rabbit polyclonal antibody immobilized latex particles.

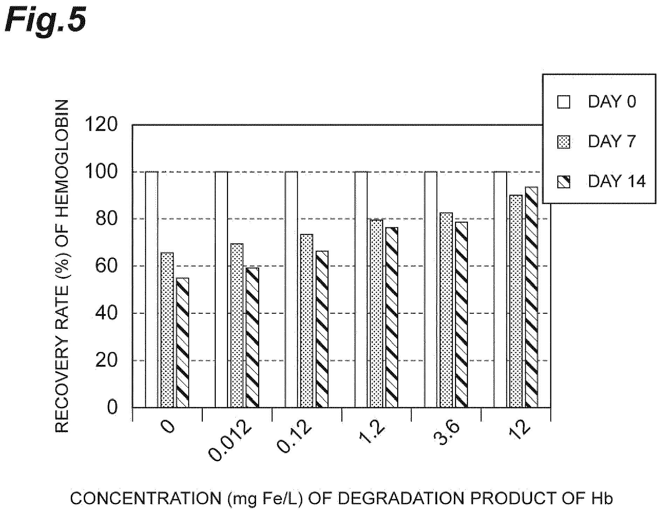

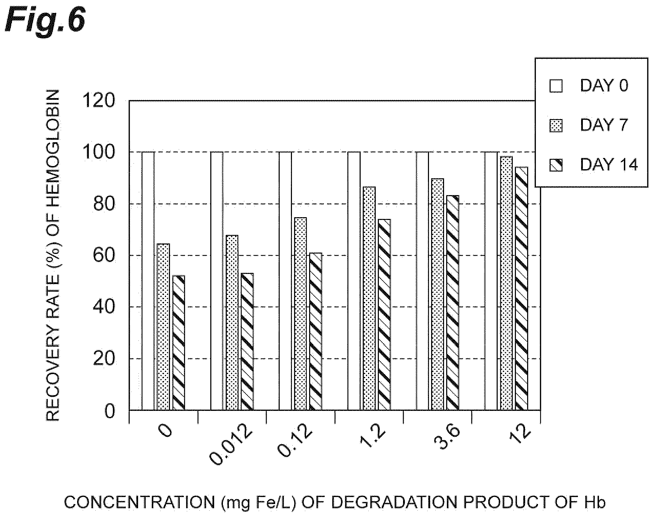

The recovery rates (%) of hemoglobin in the fecal samples were calculated from the measured concentrations of hemoglobin, based on the concentrations of hemoglobin immediately after the fecal specimens were added to the preservation solutions (that is, concentrations on day 0 after the addition of the fecal specimens). The results are shown in Table 3 and Figs. 5 and 6. Figs. 5 and 6 respectively show results of fecal samples 1 and 2. In Table 3, the concentration of an Hb degradation product is expressed in terms of iron equivalent concentration (mg Fe/L). As shown in this table and the drawings, in all of the fecal samples of the feces 1 and 2, the recovery rates of hemoglobin in the samples improved in a concentration-dependent manner due to the addition of the Hb degradation product. This result showed that the Hb degradation products stabilized hemoglobin. Since hemoglobins in the samples were present in a form of Hb-Hp complexes formed by binding to haptoglobins contained in the preservation solutions, the above-described result means that the Hb degradation products stabilized the Hb-Hp complexes.

**[Table 3]**

| Preservation temperature | Concentration (mg Fe/L) of Hb degradation product * | Concentration (µg/L) of hemoglobin | | | | | |
|---|---|---|---|---|---|---|---|
| | | Feces 1 | | | Feces 2 | | |
| | | Day 0 | Day 7 | Day 14 | Day 0 | Day 7 | Day 14 |
| 37°C | 0.012 | 512 | 356 | 303 | 527 | 357 | 279 |
| | 0.12 | 512 | 376 | 340 | 548 | 409 | 334 |
| | 1.2 | 488 | 388 | 372 | 520 | 450 | 385 |
| | 3.6 | 486 | 401 | 382 | 489 | 439 | 407 |
| | 12 | 413 | 372 | 386 | 440 | 432 | 414 |

| Preservation temperature | Concentration (mg Fe/L) of Hb degradation product * | Recovery rate (%) of hemoglobin | | | | | |
|---|---|---|---|---|---|---|---|
| | | Feces 1 | | | Feces 2 | | |
| | | Day 0 | Day 7 | Day 14 | Day 0 | Day 7 | Day 14 |
| 37°C | 0.012 | 100 | 70 | 59 | 100 | 68 | 53 |
| | 0.12 | 100 | 73 | 66 | 100 | 75 | 61 |
| | 1.2 | 100 | 80 | 76 | 100 | 87 | 74 |
| | 3.6 | 100 | 83 | 79 | 100 | 90 | 83 |
| | 12 | 100 | 90 | 93 | 100 | 98 | 94 |

| | | | | | | | |
|---|---|---|---|---|---|---|---|
| *: Concentration in terms of iron equivalent | | | | | | | |

### (Test Example 2-2)

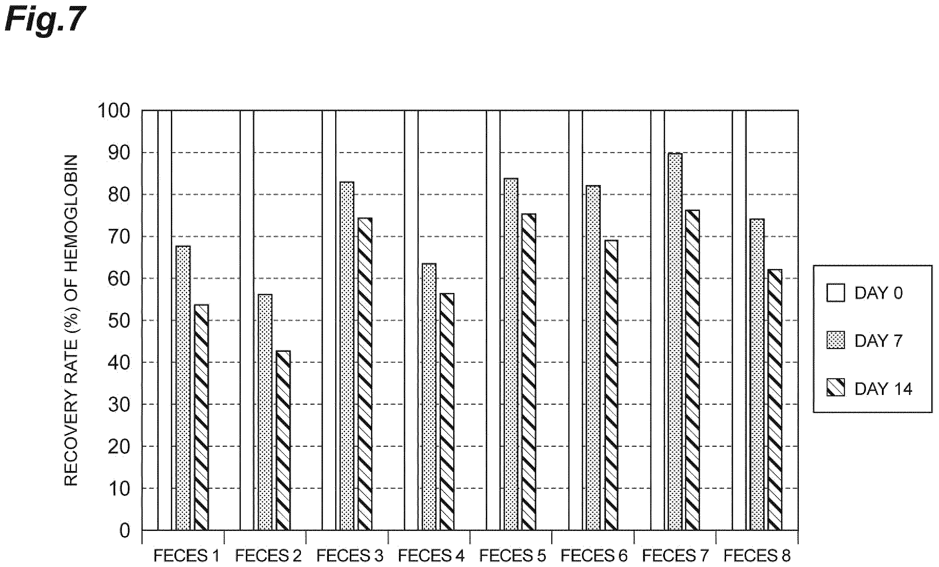

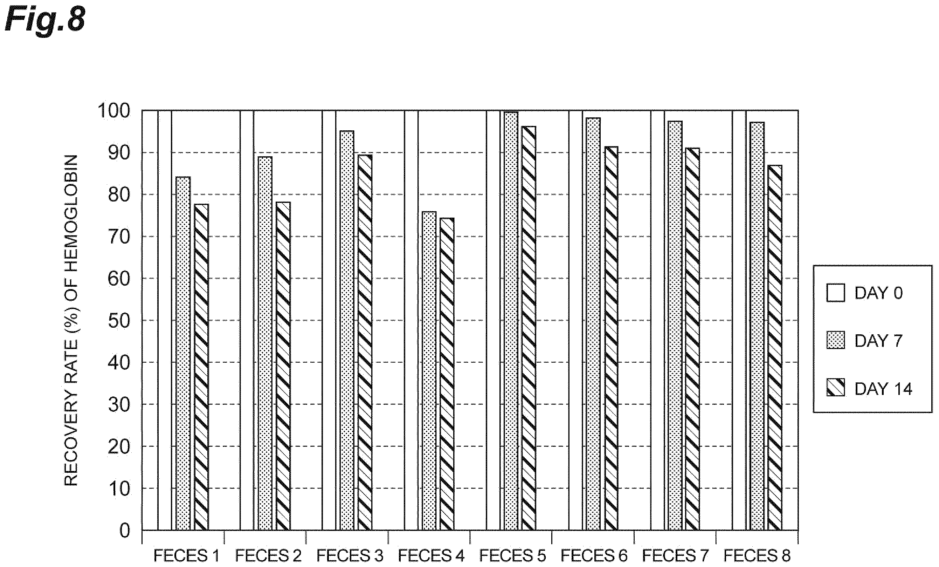

A test was performed in the same manner as in Test Example 2-1, but with the concentration of a degradation product of hemoglobin added to a preservation solution fixed to 300 mg/L (3.6 mg Fe/L of iron equivalent amount). In addition, as comparative examples, the same test was performed without adding degradation product of hemoglobin to a preservation solution. The results are shown in Table 4 and Figs. 7 and 8. Figs. 7 and 8 respectively show results of examples in which a degradation product of hemoglobin was not added and examples in which a degradation product of hemoglobin was added. As shown in this table and these drawings, the recovery rates of hemoglobin in samples improved due to the addition of the Hb degradation product. This result showed that the Hb degradation products stabilized hemoglobin. Since hemoglobins in the samples were present in a form of Hb-Hp complexes formed by binding to haptoglobins contained in the preservation solutions, the above-described result means that the Hb degradation products stabilized the Hb-Hp complexes.

**[Table 4]**

| | Preservation temperature | Concentration (µg/L) of hemoglobin | | | | | |
|---|---|---|---|---|---|---|---|
| | | Hb degradation product: not added | | | Hb degradation product: added | | |
| | | Day 0 | Day 7 | Day 14 | Day 0 | Day 7 | Day 14 |
| Feces 1 | 37°C | 514 | 348 | 276 | 482 | 405 | 374 |
| Feces 2 | | 527 | 296 | 225 | 490 | 435 | 383 |
| Feces 3 | | 487 | 404 | 362 | 495 | 469 | 442 |
| Feces 4 | | 474 | 301 | 267 | 493 | 374 | 367 |
| Feces 5 | | 485 | 407 | 366 | 505 | 502 | 485 |
| Feces 6 | | 500 | 410 | 345 | 512 | 503 | 467 |
| Feces 7 | | 480 | 430 | 366 | 501 | 488 | 455 |
| Feces 8 | | 487 | 361 | 302 | 511 | 496 | 446 |

| | Preservation temperature | Recovery rate (%) of hemoglobin | | | | | |
|---|---|---|---|---|---|---|---|
| | | Hb degradation product: not added | | | Hb degradation product: added | | |
| | | Day 0 | Day 7 | Day 14 | Day 0 | Day 7 | Day 14 |
| Feces 1 | 37°C | 100 | 68 | 54 | 100 | 84 | 78 |
| Feces 2 | | 100 | 56 | 43 | 100 | 89 | 78 |
| Feces 3 | | 100 | 83 | 74 | 100 | 95 | 89 |
| Feces 4 | | 100 | 63 | 56 | 100 | 76 | 74 |
| Feces 5 | | 100 | 84 | 75 | 100 | 99 | 96 |
| Feces 6 | | 100 | 82 | 69 | 100 | 98 | 91 |
| Feces 7 | | 100 | 90 | 76 | 100 | 97 | 91 |
| Feces 8 | | 100 | 74 | 62 | 100 | 97 | 87 |

### (Test Example 2-3)

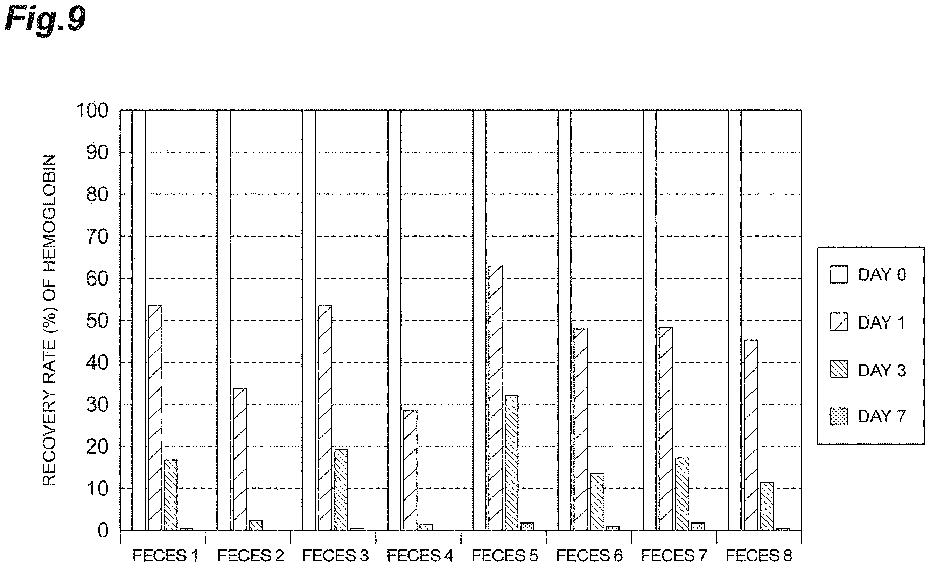

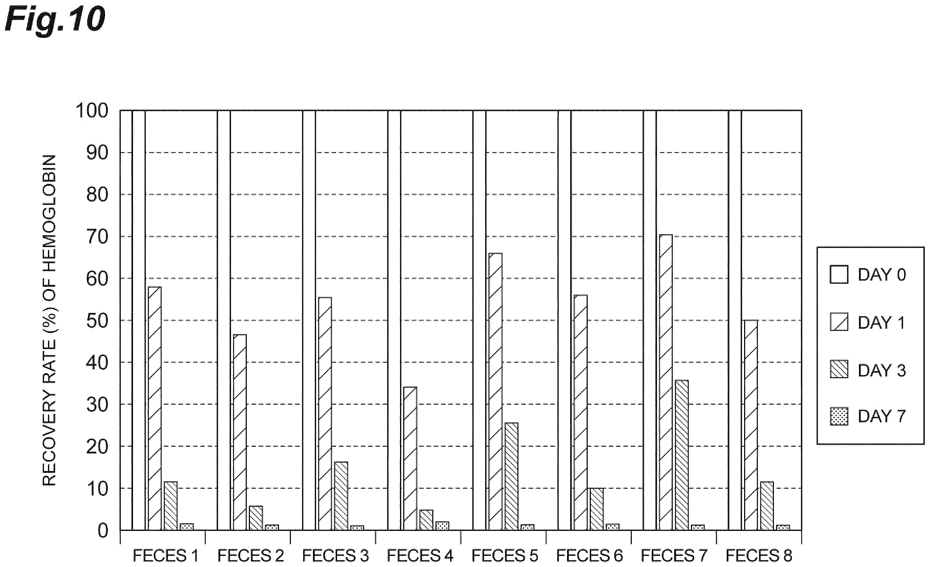

For reference, the same test as in Test Example 2-2 was performed without adding haptoglobin to preservation solutions. The results are shown in Table 5 and Figs. 9 and 10. Figs. 9 and 10 respectively show results of examples in which a degradation product of hemoglobin was not added and examples in which a degradation product of hemoglobin was added. As shown in this table and these drawings, the recovery rates of hemoglobin were low compared to the case of hemoglobin forming a complex with haptoglobin (Test Example 2-2).

**[Table 5]**

| | Preservation temperature | Concentration (µg/L) of hemoglobin | | | | | | | |
|---|---|---|---|---|---|---|---|---|---|
| | | Hb degradation product: not added | | | | Hb degradation product: added | | | |
| | | Day 0 | Day 1 | Day 3 | Day 7 | Day 0 | Day 1 | Day 3 | Day 7 |
| Feces 1 | 37°C | 489 | 263 | 82 | 1 | 479 | 279 | 55 | 9 |
| Feces 2 | | 517 | 175 | 13 | 0 | 489 | 229 | 29 | 8 |
| Feces 3 | | 528 | 283 | 102 | 2 | 510 | 284 | 83 | 6 |
| Feces 4 | | 535 | 153 | 7 | 0 | 519 | 177 | 27 | 11 |
| Feces 5 | | 529 | 334 | 170 | 8 | 525 | 347 | 136 | 8 |
| Feces 6 | | 529 | 254 | 73 | 5 | 518 | 291 | 53 | 9 |
| Feces 7 | | 498 | 241 | 86 | 8 | 509 | 359 | 182 | 7 |
| Feces 8 | | 528 | 239 | 60 | 1 | 512 | 256 | 59 | 6 |

| | Preservation temperature | Recovery rate (%) of hemoglobin | | | | | | | |
|---|---|---|---|---|---|---|---|---|---|
| | | Hb degradation product: not added | | | | Hb degradation product: added | | | |
| | | Day 0 | Day 1 | Day 3 | Day 7 | Day 0 | Day 1 | Day 3 | Day 7 |
| Feces 1 | 37°C | 100 | 54 | 17 | 0 | 100 | 58 | 11 | 2 |
| Feces 2 | | 100 | 34 | 3 | 0 | 100 | 47 | 6 | 2 |
| Feces 3 | | 100 | 54 | 19 | 0 | 100 | 56 | 16 | 1 |
| Feces 4 | | 100 | 29 | 1 | 0 | 100 | 34 | 5 | 2 |
| Feces 5 | | 100 | 63 | 32 | 1 | 100 | 66 | 26 | 2 |
| Feces 6 | | 100 | 48 | 14 | 1 | 100 | 56 | 10 | 2 |
| Feces 7 | | 100 | 48 | 17 | 2 | 100 | 71 | 36 | 1 |
| Feces 8 | | 100 | 45 | 11 | 0 | 100 | 50 | 12 | 1 |

## Claims

1. A method for stabilizing a hemoglobin-haptoglobin complex, the method comprising:
preserving the hemoglobin-haptoglobin complex in the presence of a degradation product of hemoglobin.

2. The method according to claim 1,
wherein the degradation product of hemoglobin is an enzymatic degradation product of hemoglobin.

3. The method according to claim 1 or 2, the method comprising:
preserving the hemoglobin-haptoglobin complex in a preservation solution comprising the degradation product of hemoglobin,
wherein a concentration of the degradation product of hemoglobin in terms of iron equivalent in the preservation solution is 0.012 mg/L or more.

4. The method according to any one of claims 1 to 3,
wherein the hemoglobin-haptoglobin complex comprises a hemoglobin-haptoglobin complex formed by bringing a specimen comprising hemoglobin into contact with haptoglobin.

5. The method according to claim 4,
wherein the specimen is feces, saliva, or urine.

6. A preservation solution for preserving a hemoglobin-haptoglobin complex, the preservation solution comprising:
a degradation product of hemoglobin.

7. A preservation solution for preserving a specimen comprising hemoglobin, the preservation solution comprising:
haptoglobin; and
a degradation product of hemoglobin.

8. The preservation solution according to claim 7,
wherein the specimen is feces, saliva, or urine.

9. The preservation solution according to claim 6, further comprising:
the hemoglobin-haptoglobin complex,
wherein the preservation solution is used as a calibrator or a control.

10. The preservation solution according to any one of claims 6 to 9,
wherein the degradation product of hemoglobin is an enzymatic degradation product of hemoglobin.

11. The preservation solution according to any one of claims 6 to 10,
wherein a concentration of the degradation product of hemoglobin in terms of iron equivalent is 0.012 mg/L or more.

12. A method for detecting hemoglobin in a specimen, the method comprising:
adding the specimen to the preservation solution according to claim 7 or 8 to obtain a sample comprising the specimen; and
detecting hemoglobin in the sample by an immunological method,
wherein hemoglobin in the sample is forming a complex with haptoglobin.

13. A kit for detecting hemoglobin in a specimen, the kit comprising:
the preservation solution according to claim 7 or 8; and
a reagent comprising an anti-hemoglobin antibody.
